# EUROPEAN PATENT SPECIFICATION

(11) **EP 3 937 799 B1**
(45) Date of publication and mention of the grant of the patent: **06.05.2026**
(21) Application number: 20717046.5
(22) Date of filing: 12.03.2020
(51) Int. Cl.: A61B 17/132

(54) **TISSUE COMPRESSION DEVICE**
GEWEBEKOMPRESSIONSVORRICHTUNG
DISPOSITIF DE COMPRESSION DE TISSU

(30) Priority: 14.03.2019 US 201962818530 P; 11.03.2020 US 202016816235
(43) Date of publication of application: 19.01.2022
(73) Proprietor: Medtronic Vascular, Inc., Santa Rosa, CA 95403 (US)
(72) Inventor: CASTELLI, Brian, Santa Rosa, California 95403 (US)
(74) Representative: Zimmermann & Partner Patentanwälte mbB
(86) International application number: PCT/US2020/022243
(87) International publication number: WO 2020/185992

(56) References cited:
- EP-A1- 1 295 564
- EP-A1- 3 345 555
- EP-A1- 3 345 556
- EP-A1- 3 360 471
- WO-A1-2017/165108
- CN-U- 201 551 360
- CN-U- 204 379 363
- US-A- 5 468 220
- US-A1- 2018 014 832

## Description

### TECHNICAL FIELD

This disclosure relates to tissue compression devices.

### BACKGROUND

A medical catheter may be advanced through an access site into vasculature of a patient to provide a lumen through which a medical device or a therapeutic agent may be introduced to reach a treatment site. For example, the access site for percutaneous coronary procedures may include the radial artery or the femoral artery of a patient. CN 201 551 360 U describes a radial artery extracorporeal compression apparatus. US 5,468,220 A relates to a carpal tunnel bracelet. EP 1 295 564 A1 describes an adjustable radial artery compressor. A further non-circumferential tissue compressing device is known from CN204379363U.

### SUMMARY

The present disclosure describes tissue compression devices and techniques to apply pressure to an ulnar region (e.g., and not a radial region), a radial region (e.g., and not an ulnar region), or both the ulnar and radial regions of a patient before, during, and/or after a transradial access procedure. The ulnar region may include tissue at or near (e.g., superficial to) an ulnar artery and/or ulnar veins in an arm of the patient. The radial region may include tissue at or near (e.g., superficial to) a radial artery and/or radial veins in an arm of the patient. Application of pressure at the ulnar region may be used to facilitate transradial access, reduce occlusion of the radial artery after transradial procedure, or both. Application of pressure at the radial region may be used to facilitate transradial access, achieve patent hemostasis of the access site of the radial artery after transradial procedure, or both.

According to the invention, which relates to a tissue compression device as defined in the independent claim 1, the tissue compression device is non-circumferential, which enables it to apply pressure to less than the entire outer perimeter of a wrist of a patient at a particular time. In this way, the tissue compression device may be configured to more selectively (e.g., more directly) apply pressure to only one of the ulnar region or the radial region at a time. The non-circumferential tissue compression device includes a base configured to, when on a wrist of a patient, surround at least a portion of the wrist in a non-circumferential configuration, and an expandable member coupled to the base. The non-circumferential configuration may include a "C" shape configured to allow a clinician to fit the tissue compression device onto the wrist of the patient, orient the tissue compression device to engage the wrist of the patient, and to enable the expandable member to be inflated without becoming disengaged with the wrist of the patient. The expandable member is configured to be positioned over an ulnar region or a radial region of the patient when the wrist is engaged. The expandable member also is configured to apply pressure to the ulnar region or radial region before, during, and/or after a transradial access procedure.

According to the invention the device further comprises an expandable member mechanically connected to the base. The expandable member is configured to be positioned over an ulnar region or a radial region of the wrist of the patient when the base is engaged with the portion of the wrist. The expandable member also is configured to apply pressure to the ulnar region or the radial region.

In some aspects of the tissue compression device, the expandable member comprises a bladder configured to inflate to at least a pressure to cause compression of tissue near at least one of an ulnar artery or a radial artery of the patient.

In some aspects the tissue compression device, when on the wrist of the patient, the tissue compression device is configured to partially surround the wrist in a first orientation and in a second, different orientation, and, when in the first orientation, the expandable member is positioned over the ulnar region, and, when in the second orientation, the expandable member is positioned over the radial region.

In some aspects the tissue compression device, when the base is on the wrist of the patient, the expandable member is configured to more directly apply pressure to the ulnar region than the radial region.

In some aspects of the tissue compression device, when the base is on the wrist of the patient, the expandable member is configured to more directly apply pressure to the radial region than the ulnar region.

In some aspects of the tissue compression device, the base comprises a first curved section extending from a terminal end to a medial end, wherein the first curved section is configured to traverse one of a radial side of the wrist or an ulnar side of the wrist when the base is on the wrist, a second curved section extending from a terminal end to a medial end, wherein the second curved section is configured to traverse the other of the radial side of the wrist or the ulnar side of the wrist when the base is on the wrist, and a substantially straight section extending between the medial end of the first curved section and the medial end of the second curved section. In some aspects of the tissue compression device, the substantially straight section is configured to traverse a dorsal surface of the wrist when the base is on the wrist. In some aspects of the tissue compression device, the first curved section is configured to extend from the dorsal surface of the wrist to a palmar surface of the wrist when the base is on the wrist. In some aspects of the tissue compression device, the second curved section extends from the dorsal surface of the wrist to traverse at least a portion of one of a radial border of the wrist or an ulnar border of the wrist when the base is on the wrist. In some aspects of the tissue compression device, the terminal end of the first curved section and the terminal end of the second curved section define a gap, wherein the gap is sized to enable the wrist of the patient to pass through the gap.

In some aspects of the tissue compression device, the base is configured to engage a left wrist of the patient, a right wrist of the patient, or both the left wrist and the right wrist of the patient.

In some aspects of the tissue compression, the expandable member is adjustably mechanically connected to the base.

According to the invention, the expandable member comprises a protrusion extending from a surface of the expandable member and configured to apply pressure to a tissue near an ulnar artery or a radial artery of the patient.

In some aspects of the tissue compression device, the expandable member is a first expandable member configured to be positioned over the ulnar region of the wrist of the patient when the base surrounds the portion of the wrist and apply pressure to the ulnar region, wherein the tissue compression device further comprises a second expandable member mechanically connected to the base, wherein the second expandable member is configured to be positioned over the radial region of the wrist of the patient and apply pressure to the radial region when the base surrounds the portion of the wrist. In some aspects of the tissue compression device, the first expandable member comprises a first bladder configured to inflate to at least a pressure to cause compression of tissue near an ulnar artery of the patient, and the second expandable member comprises a second bladder configured to inflate to at least a pressure to cause patent hemostasis of a vascular access site at the radial artery of the patient. In some aspects of the tissue compression device, the second expandable member comprises a bladder configured to inflate to at least a pressure to urge the wrist of the patient toward the first expandable member.

In some aspects of the tissue compression device, the base comprises a substantially rigid thermoplastic or a substantially rigid thermoset plastic.

In some aspects of the tissue compression device, when the base is on the wrist of the patient, the expandable member is configured to more directly apply pressure to the ulnar region than the radial region. In some aspects of the tissue compression device, when the base is on the wrist of the patient, the expandable member is configured to more directly apply pressure to the radial region than the ulnar region.

According to the invention, the base comprises a first section extending from a terminal end to a medial end, wherein the first section is configured to traverse one of a radial side of the wrist or an ulnar side of the wrist when the base is on the wrist, a second section extending from a terminal end to a medial end, wherein the second section is configured to traverse the other of the radial side of the wrist or the ulnar side of the wrist when the base is on the wrist, and a third section extending between the medial end of the first section and the medial end of the second section. In a preferred embodiment the terminal end of the first section and the terminal end of the second section define a gap, wherein the gap is sized to enable the wrist of the patient to pass through the gap.

In some aspects of the tissue compression device, the terminal end of the first curved section and the terminal end of the second curved section define a gap, wherein the gap is sized to enable the wrist of the patient to pass through the gap.

In some aspects of the tissue compression device, the expandable member comprises a protrusion extending from the surface of the expandable member and configured to apply pressure to a tissue near an ulnar artery or a radial artery of the patient.

In some aspects of the tissue compression device, the expandable member is a first expandable member configured to be positioned over the ulnar region of the wrist of the patient when the base is on the wrist and apply pressure to the ulnar region, wherein the tissue compression device further comprises a second expandable member mechanically connected to the base, wherein the second expandable member is configured to be positioned over the radial region of the wrist of the patient when the base is on the wrist and apply pressure to the radial region.

In some examples not part of the present invention, a method of using a tissue compression device may include positioning the tissue compression device on a wrist of a patient such that a base of the tissue compression device surrounds at least a portion of an outer perimeter of the wrist in a non-circumferential manner, and such that an expandable member mechanically connected to the base is positioned over an ulnar region or a radial region of the wrist of the patient. The method also may include inflating the expandable member to apply pressure to the ulnar region or the radial region.

In some aspects of the method, the expandable member comprises a bladder, and inflating the expandable member comprises inflating the bladder to at least a pressure to cause compression of tissue near at least one of an ulnar artery or a radial artery of the patient.

In some aspects of the method, the tissue compression device is configured to partially surround the wrist when on the wrist of the patient in a first orientation and when on the wrist of the patient in a second, different orientation, and the step of positioning the tissue compression device on the wrist of the patient comprises positioning the tissue compression device in the first orientation such that the expandable member is positioned over the ulnar region, or the step of positioning the tissue compression device on the wrist of the patient comprises positioning the tissue compression device in the second orientation such that the expandable member is positioned over the radial region.

In some aspects of the method, positioning the tissue compression device on the wrist of the patient further comprises positioning the expandable member to apply pressure more directly to the ulnar region than to the radial region.

In some aspects of the method, positioning the tissue compression device on the wrist of the patient further comprises positioning the expandable member to apply pressure more directly to the radial region than to the ulnar region.

In some aspects of the method, the base comprises a substantially rigid thermoplastic or a substantially rigid thermoset plastic that resists deformation of the base when inflating the expandable member.

In some aspects of the method of, the base comprises a first section extending from a terminal end to a medial end, wherein positioning the tissue compression device comprises traversing, with the first section, one of a radial side of the wrist or an ulnar side of the wrist, a second section extending from a terminal end to a medial end, wherein positioning the tissue compression device comprises traversing, with the second section, the other of the radial side of the wrist or the ulnar side of the wrist when the base is on the wrist, and a third section extending between the medial end of the first section and the medial end of the second section.

In some aspects of the method, positioning the tissue compression device comprises the third section traversing a dorsal surface of the wrist when the base is on the wrist. In some aspects of the method, positioning the tissue compression device on a wrist of a patient comprises positioning the first section to extend from the dorsal surface of the wrist to a palmar surface of the wrist and positioning the second section to extend from the dorsal surface of the wrist to traverse at least a portion of one of a radial border of the wrist or an ulnar border of the wrist.

In some aspects of the method, the terminal end of the first section and the terminal end of the second section define a gap, and positioning the tissue compression device on the wrist of the patient comprises passing the wrist of the patient through the gap.

In some aspects of the method, positioning the tissue compression device comprises positioning the tissue compression device on a left wrist of the patient or a right wrist of the patient.

In some aspects of the method, the expandable member is adjustably mechanically connected to the base, and the method comprises, before inflating the expandable member, adjusting a position of the expandable member relative to the base.

In some aspects of the method, the expandable member comprises a protrusion extending from a surface of the expandable member, and positioning the tissue compression device on a wrist of a patient comprises positioning the protrusion to apply pressure to a tissue near an ulnar artery or a radial artery of the patient.

In some aspects of the method, the expandable member is a first expandable member, and the tissue compression device further comprises a second expandable member mechanically connected to the base, and positioning the tissue compression device on a wrist of a patient comprises positioning the first expandable member over the ulnar region of the wrist of the patient to apply pressure to the ulnar region when the base engages at least the portion of the wrist and positioning the second expandable member over the radial region of the wrist of the patient when the base engages at least the portion of the wrist. The method further includes inflating the first expandable member to apply pressure to the ulnar region, and inflating the second expandable member to apply pressure to the radial region.

In some aspects of the method, the first expandable member comprises a first bladder and the second expandable member comprises a second bladder, wherein inflating the first expandable member comprises inflating the first bladder to at least a pressure to cause compression of tissue near an ulnar artery of the patient, and wherein inflating the second expandable member comprises inflating the second bladder to at least a pressure to cause patent hemostasis of a vascular access site at the radial artery of the patient.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a conceptual perspective view of an example non-circumferential tissue compression device including a base and an expandable member.
FIGS. 2A-2D are conceptual perspective views of a non-circumferential tissue compression device according to the invention, the device including a base and an expandable member having a protrusion.
FIGS. 3A-3E are conceptual perspective views of an example non-circumferential tissue compression device including a base and two expandable members.
FIG. 4 is a flow diagram illustrating an example method of assembling a non-circumferential tissue compression device including a base and an expandable member.
FIG. 5 is a flow diagram illustrating an example method of using a non-circumferential tissue compression device including a base and an expandable member.

### DETAILED DESCRIPTION

Percutaneous coronary procedures with transradial access include accessing vasculature of a patient via an access site, e.g., radial artery access site. The amount of blood flow in the radial artery may affect the ease of access to the radial artery or, once in the radial artery, access through the radial artery to other sites in the vasculature. For example, it is believed that increased blood flow in the radial artery may increase a diameter of the radial artery, and a larger diameter radial artery may enable easier navigation of a guide device (e.g., a guidewire or a guide catheter) or other medical device through the radial artery to a treatment site in the patient. Blood flow in the radial artery may be increased by compression of the ulnar artery, which may facilitate access of the radial artery.

The disclosure describes non-circumferential tissue compression devices configured to apply pressure to an ulnar region, a radial region, or both the ulnar and radial regions of a patient, as well as techniques for forming and using the non-circumferential tissue compression devices. The ulnar region may include selected tissue near (e.g., superficial to) an ulnar artery and/or an ulnar vein of the patient. The radial region may include selected tissue near (e.g., superficial to) a radial artery and/or a radial vein of the patient. Example non-circumferential tissue compression devices described herein are configured to apply pressure to tissue at the ulnar region, a radial region, or both to compress at least one of the ulnar artery, the ulnar vein, the radial artery, or the radial vein.

In contrast to some circumferential tissue compression devices, the non-circumferential tissue compression devices described herein are configured to apply pressure to less than the entire outer perimeter of a wrist of a patient at a particular time. In this way, the tissue compression device may be configured to more selectively (e.g., more directly) apply pressure to only one of the ulnar region or the radial region at a time. For example, the tissue compression device may be configured to apply pressure to only one of the ulnar region or the radial region while not applying pressure to the other of the ulnar region or the radial region or while applying substantially less pressure to the other of the ulnar region or the radial region. The substantially less pressure can be, for example, pressure that does not result in a physiologically significant change to blood flow in the one of the ulnar region or the radial region.

The non-circumferential tissue compression device includes a base and an expandable member mechanically connected to the base. The base includes a surface configured to, when on the wrist of the patient, surrounds or engages a portion of the wrist of the patient in a non-circumferential configuration. For example, the surface may surround or engage with less than a full outer perimeter of the wrist of the patient. The non-circumferential configuration may include a "C" shape configured to allow a clinician to fit the tissue compression device onto the wrist of the patient, orient the tissue compression device to engage the wrist of the patient, and to enable the expandable member to be inflated without becoming disengaged with the wrist of the patient. For example, the base and/or base surface may define an arc extending between greater than about 180 degrees to less than 360 degrees.

In some examples, the expandable member may include a single expandable chamber, e.g., balloon. A single expandable member may be configured to apply pressure to the ulnar region, the radial region, or both regions simultaneously. In some examples, the expandable member may include a plurality of expandable members. The plurality of expandable members may be configured to individually or collectively apply pressure to the ulnar region, the radial region, or both regions simultaneously. In some examples, the non-circumferential tissue compression device may be reversible such that in a first orientation the expandable member may be oriented over the ulnar region and not over the radial region, and in a second, different (e.g., reversed) orientation the expandable member may be oriented over the radial region and not over the ulnar region.

In this way, the tissue compression device is configured to apply pressure to selected tissue beneath the expandable member, e.g., to tissue that transmits the pressure to at least one of the ulnar artery, ulnar vein, the radial artery, or the radial vein. It is believed that ulnar artery compression may improve the outcomes or ease of transradial percutaneous coronary procedures by facilitating access to the vasculature of the patient. For example, a weak radial pulse due to a relatively small radial artery, a relatively deep radial artery, or hypotension may make vasculature access by arteriotomy of the radial artery difficult. It is believed that ulnar compression may be used to improve accuracy and speed of vasculature access via arteriotomy of the radial artery by making radial pulse stronger and/or enlarging the radial artery to improve success rate of fingertip palpitation-guided access to the radial artery.

In some cases, pressure applied to the access site after the procedure to achieve patent hemostasis may result in radial artery occlusion (e.g., acute and/or chronic radial artery occlusion). Radial artery occlusion may be caused by acute thrombosis due to intimal abrasion and changes in the vessel in conjunction with blood flow cessation when pressure is applied to the radial artery access site (e.g., radial compression) to achieve hemostasis. Radial artery occlusion may be undesirable. For example, radial artery occlusion may limit future ipsilateral transradial access, cause transient discomfort to the patient, or any combination thereof.

It is believed that ipsilateral ulnar artery compression (also referred to as ulnar compression) may maintain or increase radial artery blood flow to facilitate access of the radial artery or, during radial compression, to reduce incidence of radial artery occlusion compared to other methods alone, such as compared to the use of a low sheath (part of the percutaneous access device) to artery size ratio, the use of intraprocedural heparin, or the maintenance of radial artery patency during hemostasis after transradial access. Ulnar compression may result in complete or near complete occlusion of the ulnar artery to increase blood flow in the radial artery. The increased blood flow in the radial artery may enable compression of the radial artery with a pressure sufficient to achieve hemostasis at the transradial access site without causing radial artery occlusion.

While circular tissue compression devices are useful, the non-circumferential tissue compression devices described herein are configured to more selectively apply pressure to one of the ulnar region or the radial region, or both regions simultaneously, which may facilitate radial artery access, shorten the time to achieve patent hemostasis, reduce incidence of radial artery occlusion, improve the healing process after percutaneous coronary procedures with transradial access, and improve patient comfort compared to other methods, such as tissue compression with a circumferential tissue compression band.

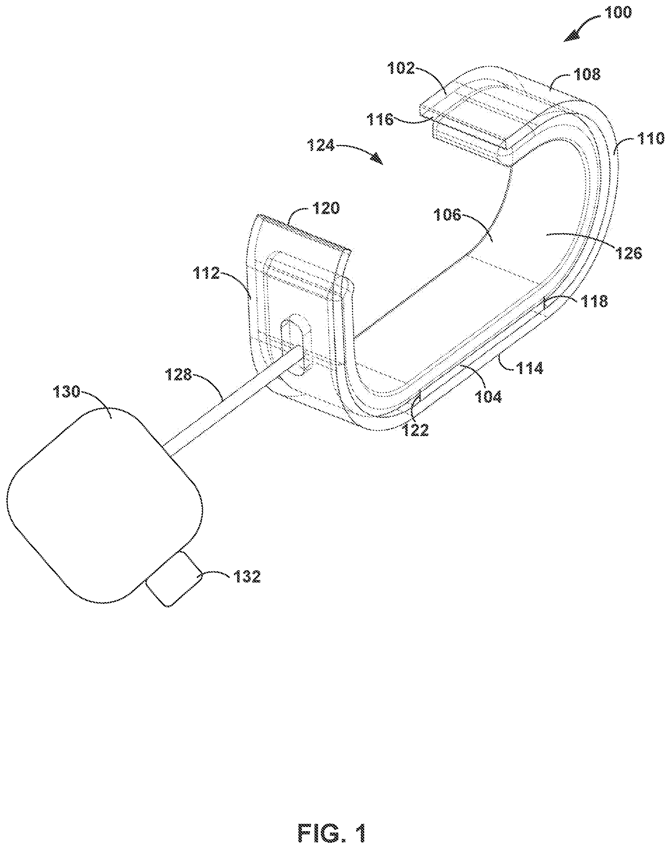

FIG. 1 is a conceptual perspective view of an example non-circumferential tissue compression device 100 including a base 102 and an expandable member 106. Non-circumferential tissue compression device 100 is configured to receive and engage a wrist of a patient to apply pressure to selected tissue at the ulnar region, the radial region, or both the ulnar region and the radial region (e.g., aligned with but superficial to at least one of the ulnar artery, ulnar vein, the radial artery, or the radial vein) of the patient to thereby apply pressure to the respective at least one of the ulnar artery ulnar vein, the radial artery, or the radial vein. Base 102 is configured to partially surround a wrist of a patent and not fully surround the wrist, e.g., as in a bracelet type configuration. For example, base 102 includes a first surface 104 configured to, when on the wrist of the patient, surround or engage at least a portion of the wrist in a non-circumferential configuration. Base 102 and/or first surface 104 may, therefore, be referred to as a non-circumferential base 102 or a non-circumferential surface 104, respectively. Base 102 also defines a second surface 108 on an opposite side of base 102 from surface 104. Surfaces 104, 108 face in different directions.

First surface 104 may have any suitable shape configured to engage with at least a portion of the wrist and/or forearm of the patient. For example, base 102 and/or first surface 104 may have a non-circumferential "C" shape that corresponds to an anatomical shape of at least part of the wrist of a patient (e.g., mimics the general outline of the wrist or part of the wrist, such as the dorsal surface of the wrist, the ulnar side of the wrist (e.g., ulnar border), and the radial side of the wrist (e.g., radial border). The non-circumferential "C" shape may define an arc extending between greater than about 180 degrees to less than 360 degrees, such as, for example, about 200 degrees to about 320 degrees or about 270 degrees. By extending greater than about 180 degrees, a force may be applied by expandable member 106 to the wrist of the patient at a first end (e.g., terminal end 116) of non-circumferential tissue compression device 100, and the force may be counteracted at least at a second end (e.g., terminal end 120) of non-circumferential tissue compression device 100 such that the expandable member 106 compresses tissue beneath expandable member 106, rather than dislodging non-circumferential tissue compression device 100 from the wrist of the patient.

In addition, by extending less than about 360 degrees, non-circumferential base 102 may define a gap 124 configured to enable a clinician to fit non-circumferential tissue compression device 100 to a wrist of the patient. In some examples, first surface 104 may have a shape that corresponds to an anatomical shape of both the right hand and the left hand of the patient, e.g., first surface 104 may be ambidextrous. In other examples, first surface 104 may have other shapes, such as elliptical, rectangular, or irregular shapes. In this way, the shape of first surface 104 may be selected to allow a clinician to fit base 102 onto the wrist of the patient, orient the tissue compression device 100 to engage the wrist of the patient, and to enable expandable member 106 to be inflated without becoming disengaged with the wrist of the patient

In some examples, base 102 may include a plurality of connected sections or segments. For example, base 102 may include a first curved section 110, a second curved section 112, and a substantially straight section 114. First curved section 110 and second curved section 112 are each example structures configured to hold non-circumferential tissue compression device 100 on the wrist of the patient when expandable member 106 is inflated. First curved section 110 extends from a terminal end 116 to a medial end 118. First curved section 110 is configured to extend around or engage one of a radial side of the wrist or an ulnar side of the wrist. For example, when the wrist is engaged with first surface 104, first curved section 110 may extend from the dorsal surface of the wrist to a palmar surface of the wrist (e.g., traversing the ulnar border of the wrist or the radial border of the wrist) to surround or engage at least a portion of the ulnar border of the wrist or the radial border of the wrist. By surrounding or engaging at least a portion of the ulnar border of the wrist or the radial border of the wrist, first curved section 110 may reduce movement of non-circumferential tissue compression device 100 when expandable member 106 is inflated to prevent dislodgement of base 102 from the wrist of the patient.

Second curved section 112 extends from a terminal end 120 to a medial end 122. Second curved section 112 is configured to surround or engage the other of the radial side of the wrist or the ulnar side of the wrist. For example, second curved section 112 may extend from the dorsal surface of the wrist to traverse at least a portion of one of the radial border of the wrist or the ulnar border of the wrist be configured to engage at least a portion of the radial border of the wrist of the patient when the wrist is engaged with first surface 104. By surrounding or engaging at least a portion of the ulnar border of the wrist or the radial border of the wrist, second curved section 112 may reduce movement of non-circumferential tissue compression device 100 when expandable member 106 is inflated to prevent dislodgement of base 102 from the wrist of the patient.

Substantially straight section 114 extends between medial end 118 of first curved section 110 and medial end 122 of second curved section 112. Substantially straight section 114 may be straight or nearly straight, e.g., compared to first curved section 110 and/or second curved section 114. By extending between first curved section 110 and second curved section 112, substantially straight section 114 may reduce movement of first curved section 110 relative to second curved section 112 to facilitate engagement of the ulnar border and radial border by the respective first curved section 110 and second curved section 112.

Terminal end 118 of first curved section 110 and terminal end 120 of second curved section 112 define a gap 124. Gap 124 is sized to enable a clinician to position non-circumferential tissue compression device 100 on the wrist of the patient. For example, gap 124 may be at least as wide as a projection of the wrist (e.g., the projection taken from the ulnar border or radial boarder), such that the wrist may pass through gap 124. In some examples, gap 124 may have a width between about 2.5 centimeters to about 6 centimeters. In some examples, terminal end 118 may contact or nearly contact terminal end 120, e.g., gap 124 may be between about 0 cm and about 2.5 cm, when not positioned on a wrist of the, and a clinician may apply a force to non-circumferential tissue compression device 100 to open gap 124 while positioning non-circumferential tissue compression device 100 on the wrist of the patient. After a portion of the wrist is received within gap 124, non-circumferential tissue compression device 100 may be rotated to surround the desired portion of the wrist, e.g., such that substantially straight section 114 traverses the dorsal surface of the wrist. In some examples, non-circumferential tissue compression device 100 may include one or more straps or claps traversing gap 124. The one or more straps or clasps may be configured to prevent non-circumferential tissue compression device 100 from falling off the wrist or moving relative to the wrist. The one or more straps or clasps may not apply pressure to the wrist of the patient or may not even contact the wrist of the patient.

In some examples, base 102 is formed from one or more substantially rigid materials. A substantially rigid material may include a material having an apparent modulus of rigidity (e.g., apparent shear modulus of elasticity) in ambient conditions sufficient to reduce deflection of base 102 when first surface 104 is positioned to surround or engage the wrist of the patient and expandable member 106 is expanded to apply pressure to the ulnar region and/or the radial region. For example, a substantially rigid material may include a material having an apparent shear modulus of at least approximately 0.1 gigapascal (GPa). In some examples, the substantially rigid material may include enough flexibility to enable a clinician to deform non-circumferential tissue compression device 100 for fitting to the wrist of the patient, e.g., to sufficiently widen gap 124 to fit over the wrist of the patient. After deforming non-circumferential tissue compression device 100 to fit around the wrist of the patient, the substantially rigid material may include enough elasticity to return, or nearly return, to the original shape. In this way, non-circumferential tissue compression device 100 may be configured to accommodate a selected range of wrist sizes, such as from patients with a less than average wrist diameter to patients with a greater than average wrist diameter. In some examples, base 102 includes one or more substantially rigid thermoplastics, such as acrylonitrile butadiene styrene (ABS), polyethylene, polycarbonate, polyamide, high impact polystyrene, polypropylene, or polyoxymethylene; or substantially rigid thermoset plastics, such as polyester, polyurethane, or epoxy resin.

Base 102 can be entirely rigid in some examples, while in other examples, base 102 may include one or more flexible regions or joints configured to improve patient comfort when non-circumferential tissue compression device 100 is retained on the wrist of the patient. For example, substantially straight section 114 may extend between a first joint region adjacent medial end 118 of first curved section 110 and a second joint region adjacent medial end 122 of second curved section 112. Each of the first and second joint regions may include a substantially flexible material configured to allow base 102 to bend or deflect at first joint region or second joint region in response to a force, such as forces pulling terminal ends 116 and 120 in opposing directions. In this way, first joint region or second joint region may enable a clinician to bend base 102 to widen gap 124 when positioning non-circumferential tissue compression device 100 on the wrist of the patient, as discussed above.

Expandable member 106 may be mechanically connected to first surface 104 of base 102. Expandable member 106 is configured to be positioned over the ulnar region (e.g., and not the radial region), the radial region (e.g., and not the ulnar region), or both the ulnar region and the radial region of the wrist of the patient when base 102 is engaged with the portion of the wrist. Expandable member 106 also is configured to apply pressure to the ulnar region and/or the radial region. For example, expandable member 106 may be configured to apply pressure to only one of the ulnar region or the radial region while not applying pressure to the other of the ulnar region or the radial region or while applying substantially less pressure to the other of the ulnar region or the radial region.

In some examples, expandable member 106 may include a pad disposed on first surface 104, e.g., along substantially straight section 114. The pad may be configured to engage with a dorsal surface of the wrist of the patient when the wrist is positioned on first surface 104 to improve patient comfort. In some examples, the pad is formed from a softer, more pliable material than base 102. For example, the pad may include any suitable material, such as silicone, rubber, polyethylene, or a thermoplastic or thermoset plastic. In some examples, the pad may include an inflatable balloon configured to adjustably control the size and/or rigidity of the pad. Adjustable control of the size and/or rigidity of the pad may enable non-circumferential tissue compression device 100 to accommodate different wrist sizes or shapes and adjust the extent to which the pad extends from first surface 104. The pad may be integrally formed with base 102 or adjustably mechanically connected to base 102 by, for example, an adhesive, a hook-and-loop fastener, or any other suitable fastener or combination of fasteners.

In some examples, expandable member 106 includes a coating or other material to increase the static friction between the wrist of the patient and expandable member 106, which may reduce any undesirable relative movement between the hand and non-circumferential tissue compression device 100.

Expandable member 106 is configured to be positioned over the ulnar region, the radial region, or both when non-circumferential tissue compression device 100 is retained on the wrist of the patient. Although illustrated in FIG. 1 as covering substantially the entire first surface 104, in other example, expandable member 106 may cover one or more portions of first surface 104, such as a portion of first surface 104 at first curved section 110 or a portion of first surface 104 at second curved section 112.

Expandable member 106 may be mechanically connected to base 102 by any suitable means, such as, for example, an adhesive, thermal bonding, welding, or a mechanical fastener, e.g., by a hook-and-loop fastener. In other examples, expandable member 106 may be integrally formed with base 102, such that a position of expandable member 106 is fixed relative to base 102. For example, base 102 may include two or more layers forming a pocket and defining expandable member 106. In some examples, expandable member 106 may be secured in place to base 102 at a location that positions expandable member over the ulnar region and/or radial region when non-circumferential tissue compression device 100 is retained on the wrist of the patient.

In some examples, expandable member 106 may be removably secured to base 102 such that a clinician may adjust the position of expandable member 106 relative to base 102. For example, expandable member 106 may be connected to base 102 via an adhesive, such as a pressure-sensitive adhesive or a removable adhesive, or a mechanical fastener, such as a hook-and-loop mechanism, and a clinician may detach expandable member 106 from base 102 by pulling expandable member 106 away from base 102 and then subsequently use the adhesive or hook-and-loop mechanism to reattach expandable member 106 to base 102 at a different location. In this way, a clinician may adjust the position of expandable member 106 relative to base 102 prior to retaining non-circumferential tissue compression device 100 on the wrist of the patient.

In some examples, base 102, expandable member 106, or both may include a transparent material or a window including a transparent material, the window positioned on base 102 to be substantially over an access site, such as a radial access site, when retained on the wrist of the patient. The transparent material may enable a clinician to visualize an access site while non-circumferential tissue compression device 100 is retained on the wrist of the patient. In contrast, if base 102 and expandable member 106 were entirely opaque, a clinician may not be able to view the access site. By visualizing the access site, the clinician may visually confirm patent hemostasis.

Expandable member 106 is configured to apply pressure to selected tissue at the ulnar region, radial region, or both when non-circumferential tissue compression device 100 is retained on the wrist of the patient to facilitate access to the vasculature of the patient, to help reduce incidence of radial artery occlusion after percutaneous coronary procedures with transradial access, or both. Expandable member 106 may define any suitable shape having any suitable surface area to apply pressure to selected tissue at the ulnar region and/or radial region. For example, a surface 126 of expandable member 106 may include the non-circumferential "C" shape as described above in reference to first surface 104 of base 102. For example, surface 126 may be configured to engage with selected tissue at the ulnar region and/or radial region of a patient. In other examples, surface 126 may define one or more similar or different geometric shapes, such as an ellipse, a quadrilateral or other polygon, or irregular shapes.

In some examples, surface 126 is configured to apply a substantially uniform pressure to tissue of a patient when expandable member 106 is inflated. In other examples, surface 126 may define one or more protrusions configured to increase pressure at a selected point when expandable member 106 is inflated. For example, a first protrusion may be positioned on surface 126 such that the protrusion is oriented over an ulnar region or a radial region when non-circumferential tissue compression device 100 is engaged with the wrist of the patient. Additionally, expandable member 106 define any suitable volume to achieve a selected pressure on the selected tissue at the ulnar region and/or radial region. For example, expandable member 106, when in a deflated configuration or an inflated configuration, may extend any suitable distance from first surface 104 of base 102 (or from surface 126 of expandable member 106).

Expandable member 106 may include one or more bladders (not shown, "the bladder") fluidically connected to a channel 128. Channel 128 may be fluidically connected to one or more inflation devices 130 configured to inflate the bladder of expandable member 106 and one or more deflation devices 132 configured to deflate the bladder of expandable member 106. For example, inflation device 130 may include a pump configured to controllably inflate the bladder of expandable member 106 or a syringe configured to controllably inflate the bladder of expandable member 106.

Deflation device 132 may include a release valve configured to deflate the bladder of expandable member 106. In some examples, a clinician may use deflation device 132 to uncontrollably and fully deflate the bladder of expandable member 106, e.g., prior to removing non-circumferential tissue compression device 100 from the hand of the patient. In other examples, deflation device 132 may more controllably deflate the bladder of expandable member 106 to compress of the ulnar region and/or radial region. For example, with respect to compression of the ulnar region prior to access at the radial artery, a clinician may inflate the bladder of expandable member 106 using inflation device 130 to a first pressure that is greater than a minimum pressure to achieve the desired compression of the ulnar region, e.g., the clinician may inflate the bladder of expandable member 106 until the clinician observes at least partial occlusion of the ulnar artery distal to the bladder of expandable member 106 or ulnar vein proximal to the bladder of expandable member 106 by, for example, palpitation, ultrasonography, pulse-oximetry, or modified Allen's test (e.g., the hand of the patient may be elevated, the hand may be clenched in a fist for about 30 seconds, pressure may be applied over the ulnar artery and the radial artery so as to occlude both arteries while the hand is clenched in the fist, the elevated hand may be subsequently unclenched, the color of the hand may be observed as blanched or pallor may be observed at the finger nails, ulnar artery pressure may be released while radial artery pressure is maintained, and the time for color to return to the hand is observed).

After inflating the bladder of expandable member 106 to the first pressure, the clinician may controllably deflate the bladder of expandable member 106 using deflation device 132 to a second pressure slightly less than the minimum pressure to achieve occlusion of the ulnar artery, e.g., the clinician may deflate the bladder of expandable member 106 until the clinician observe return ulnar artery blood flow. After deflating the bladder of expandable member 106 to the second pressure, the clinician may inflate the bladder of expandable member 106 by a predetermined volume, such as, for example, 1 cubic centimeter to 20 cubic centimeters of air, to a third pressure and confirm at least partial occlusion of the ulnar artery. By controllably deflating the bladder of expandable member 106 to the second pressure, the clinician may more accurately inflate the bladder of expandable member 106 to the third pressure using the predetermined volume to sufficiently compress the ulnar region to increase blood flow or blood pressure in the radial artery.

The bladder of expandable member 106 may be configured to inflate to at least a pressure that provides at least partial occlusion of the ulnar artery, patent hemostasis of a transradial access site, or both. In some examples, inflation of the bladder of expandable member 106 will tend to move surface 126 of expandable member 106 away from first surface 104 of base 102. When non-circumferential tissue compression device 100 is retained on the wrist of the patient, inflation of the bladder of expandable member 106 forces surface 126 of expandable member 106 against the tissue at at least one of the ulnar artery, the ulnar vein, the radial artery or the radial vein to provide compression of selected tissue in contact with surface 126 of expandable member 106. In this way, expandable member 106 may inflate to apply a selected pressure to tissue at the ulnar region and/or radial region.

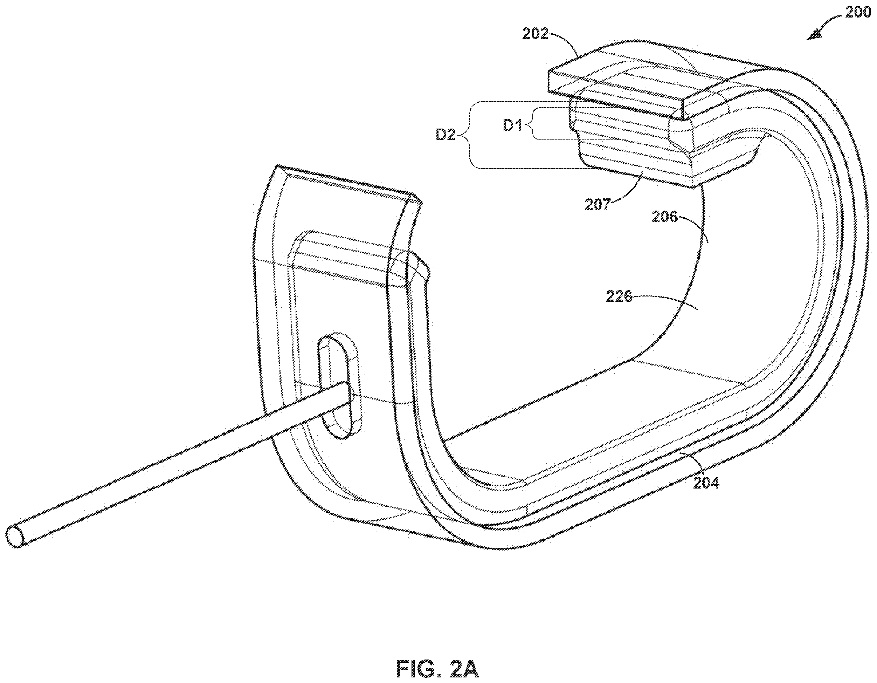

FIGS. 2A-2D are conceptual diagrams illustrating a non-circumferential tissue compression device 200 according to the invention, the device including a protrusion 207 on an expandable member 206. Non-circumferential tissue compression device 200 may be the same as or substantially similar to non-circumferential tissue compression device 100 discussed above in reference to FIG. 1, except for the differenced described herein. For example, non-circumferential tissue compression device 200 may include a non-circumferential base 202 that includes a surface 204 configured to, when on a wrist of a patient, surround or engage at least a portion of the wrist in a non-circumferential configuration, and expandable member 206 mechanically connected to surface 204 of base 202. Expandable member 206 is configured to be positioned over an ulnar region or a radial region of the wrist of the patient when base 202 is engaged with the portion of the wrist, and is configured to apply pressure to the ulnar region or the radial region.

As illustrated in FIGS. 2A-2D, expandable member 206 includes protrusion 207. Protrusion 207 is configured to apply pressure to a tissue near an ulnar artery or a radial artery of the patient. For example, when expandable member 206 is inflated, surface 226 of expandable member 206 may extend a first distance "D1" from first surface204 of base 202. Protrusion 207 may extend a second distance "D2" that is greater than D1 from first surface204 of base 202. By extending farther from first surface204 of base 202, protrusions 207 may be configured to increase pressure at a selected point beneath protrusion 207 when expandable member 206 is inflated.

Protrusion 207 may include a non-expandable protrusion, e.g., a structure fixed to surface 226, or an expandable protrusion, e.g., a structure integrally formed with expandable member 206. In examples in which protrusion 207 is a non-expandable protrusion, protrusion 207 may be adjustably mechanically connected to surface 226 by for example, an adhesive, a hook-and-loop fastener, or any other suitable fastener or combination of fasteners. In this way, a clinician may adjust a position of protrusion 207 relative to base 102 such that protrusion 207 is oriented over a selected region, such as one of ulnar region 250 or radial region 252, when base 102 is positioned on the wrist of the patient. Ulnar region 250 or radial region 252 are example regions and may vary between patients. A clinician may determine a location of ulnar region 250 or radial region 252. In examples in which protrusion 207 includes an expandable protrusion, protrusion 207 may be fluidly coupled to expandable member 206 such that increasing a pressure within expandable member 206 increases a pressure within protrusion 207 or fluidly coupled to a second inflation device and/or second deflation device, such that a pressure of protrusion 207 may be control independent of a pressure of expandable member 207. In this way, protrusion 207 may be configured to selectively apply pressure to a selected region, such as one of ulnar region 250 or radial region 252, depending on the desired goal of the application of pressure when base 102 is positioned on the wrist of the patient.

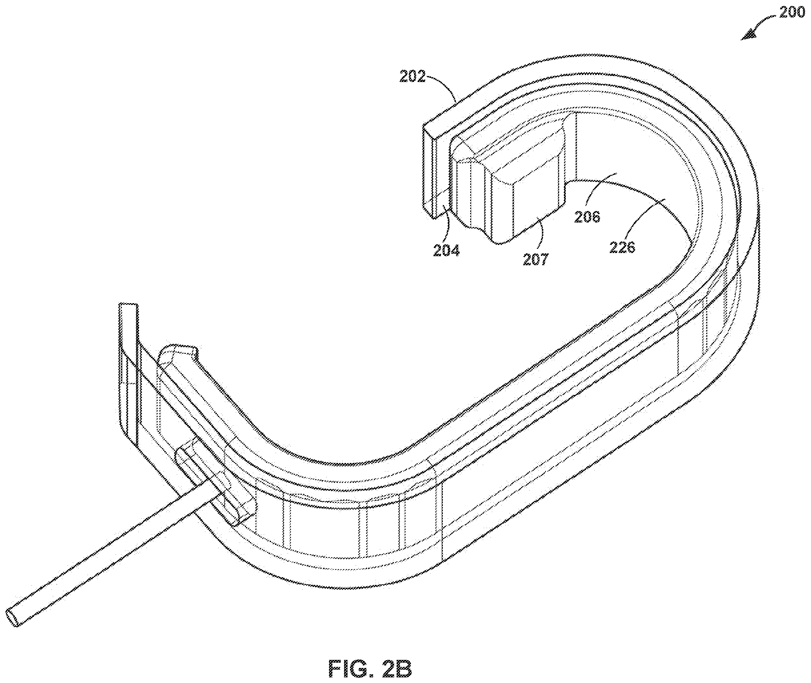

Protrusion 207 may include any suitable shape configured to increase pressure at a selected point beneath protrusion 207. As illustrated in FIG. 2B, protrusion 207 includes a tapered rectilinear shape. In other examples, protrusion 207 may include a hemispherical shape, a conical shape, or another geometric or irregular shape configured to increase pressure at a selected point beneath protrusion 207, such as shape corresponding to an anatomical shape of an ulnar region or a radial region.

In some examples, non-circumferential tissue compression device 200 may include a plurality of protrusions. For example, non-circumferential tissue compression device 200 may include a first protrusion and a second protrusion. The first protrusion may be positioned on surface 226 such that the first protrusion is oriented over an ulnar region when non-circumferential tissue compression device 200 is engaged with the wrist of the patient. The second protrusion may be positioned on surface 226 such that the second protrusion is oriented over a radial region when non-circumferential tissue compression device 200 is engaged with the wrist of the patient. In this way, a plurality of protrusions may increase pressure at a plurality of selected points beneath a respective plurality of protrusions when expandable member 206 is inflated.

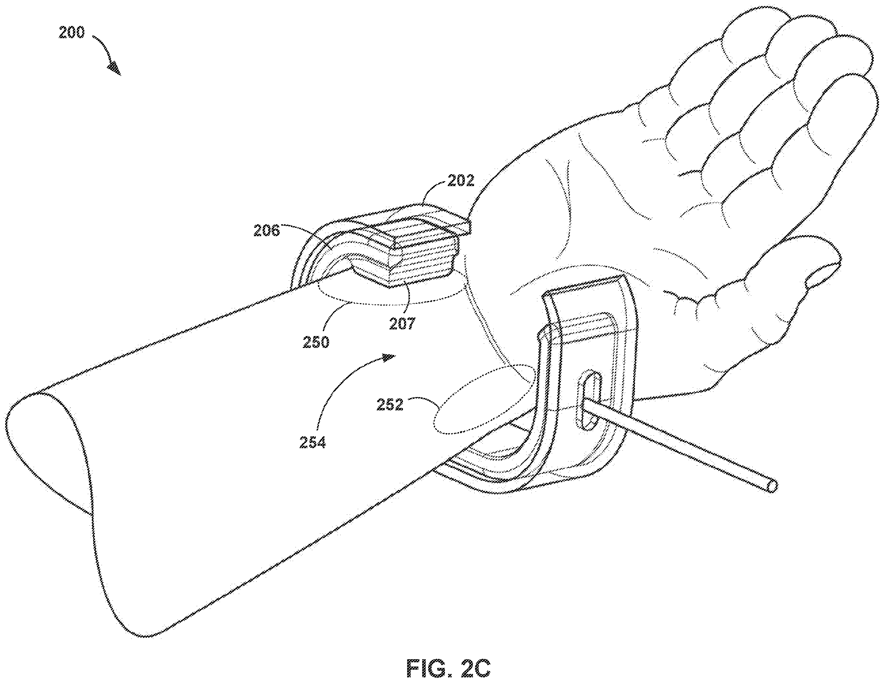

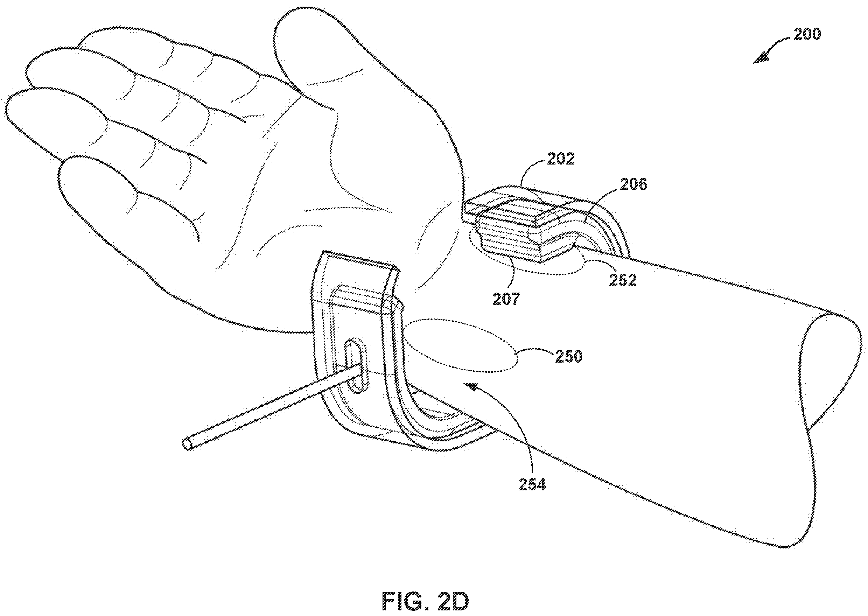

As discussed above, non-circumferential tissue compression device 200 may be reversible. For example, as illustrated in FIG. 2C, in a first orientation of tissue compression device 200, protrusion 207 may be positioned over ulnar region 250 (indicated by dashed lines) of wrist 254 of the patient and not over radial region 252 (indicated by dashed lines) of wrist 254. As illustrated in FIG. 2D, when in tissue compression device 200 is in a second, different (e.g., reversed) orientation, protrusion 207 may be positioned over radial region 252 and not over ulnar region 250. A clinician may change the orientation of non-circumferential tissue compression device 200 during a percutaneous coronary procedure. For example, the clinician may position non-circumferential tissue compression device 200 on wrist 254 of patient in the first orientation (FIG. 2C) before arteriotomy to at least partially occlude the ulnar artery which, as discussed above, may facilitate access at the radial artery. After arteriotomy, e.g., prior to application of pressure to the access site, the clinician may remove non-circumferential tissue compression device 200 for wrist 254, and refit non-circumferential tissue compression device 200 in the second orientation (FIG. 2D) which, as discussed above, may be used to apply pressure to the access site to achieve patent hemostasis of the access site. In this way, non-circumferential tissue compression device 200 may be configured to selectively apply pressure to only one of ulnar region 250 or radial region 252, depending on the desired goal of the application of pressure.

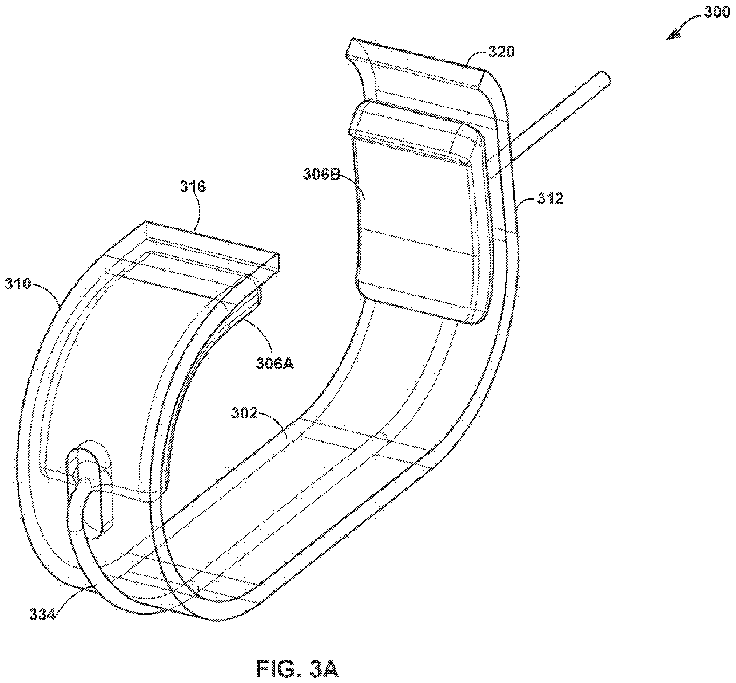

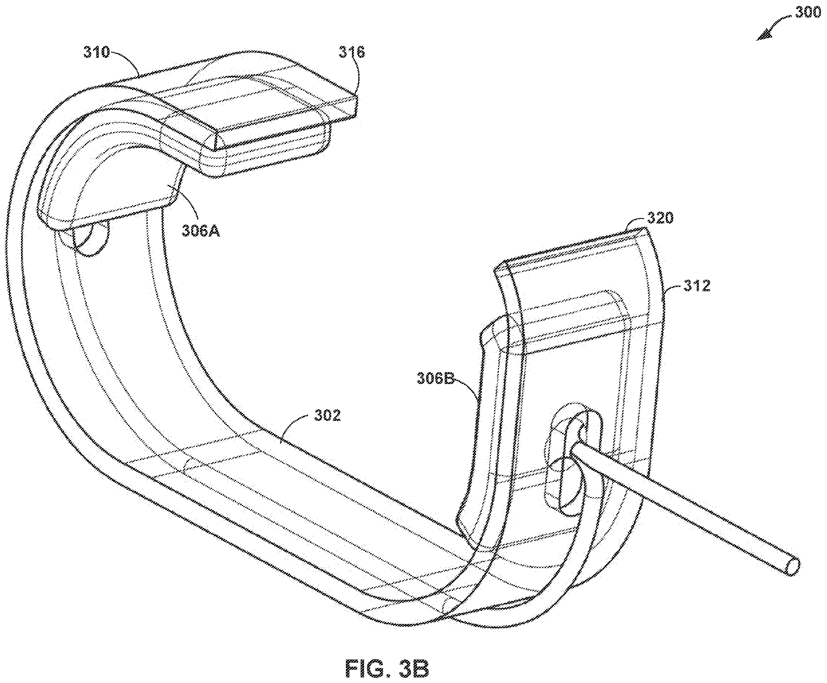

In some examples, a non-circumferential tissue compression device may include a plurality of expandable members. FIGS. 3A-3C are conceptual diagrams illustrating an example tissue compression device including a base 302 and two expandable members 306A and 306B (collectively, "expandable member 306"). Non-circumferential tissue compression device 300 may be the same as or substantially similar to non-circumferential tissue compression devices 100 and 200 discussed above in reference to FIGS. 1-2D, except for the differenced described herein. For example, non-circumferential tissue compression device 300 may include base 302 that include a surface 304 configured to, when on a wrist of a patient, surround or engage at least a portion of the wrist in a non-circumferential configuration, and expandable member 306 mechanically connected to surface 304 of base 302. Expandable member 306 is configured to be positioned over an ulnar region or a radial region of the wrist of the patient when base 302 is engaged with the portion of the wrist, and is configured to apply pressure to the ulnar region or the radial region.

As illustrated in FIGS. 3A-3D, expandable members 306A and 306B may be positioned near opposite ends of base 302. For example, first expandable member 306A may be positioned near terminal end 316 of first curved section 310. Second expandable member 306B may be positioned near terminal end 320 of second curved section 312. Positioning expandable member 306 near respective terminal ends 316 and 320 may enable a clinician to position non-circumferential tissue compression device 300 on the wrist of the patient such that expandable members 306 are oriented over a respective ulnar region or radial region.

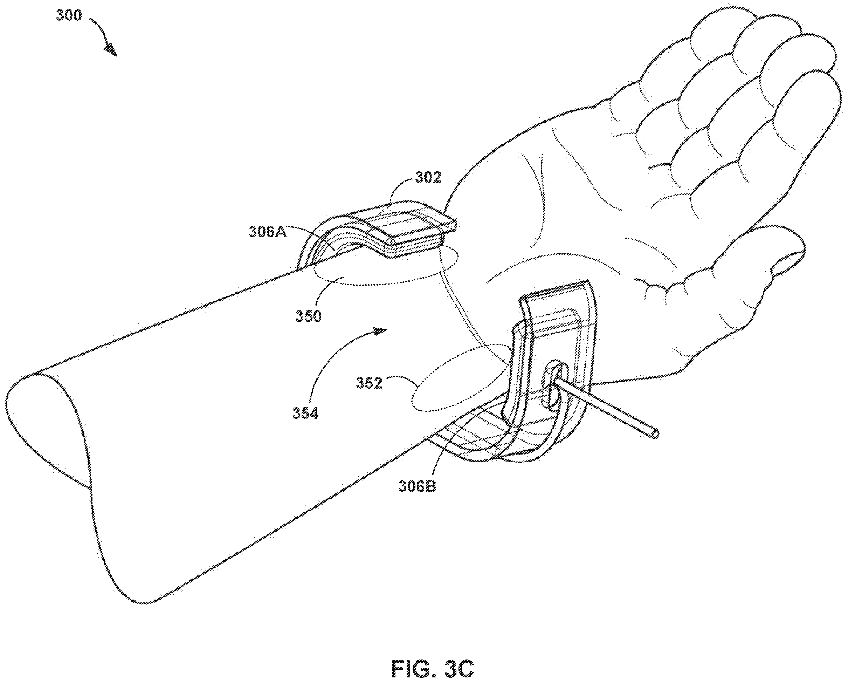

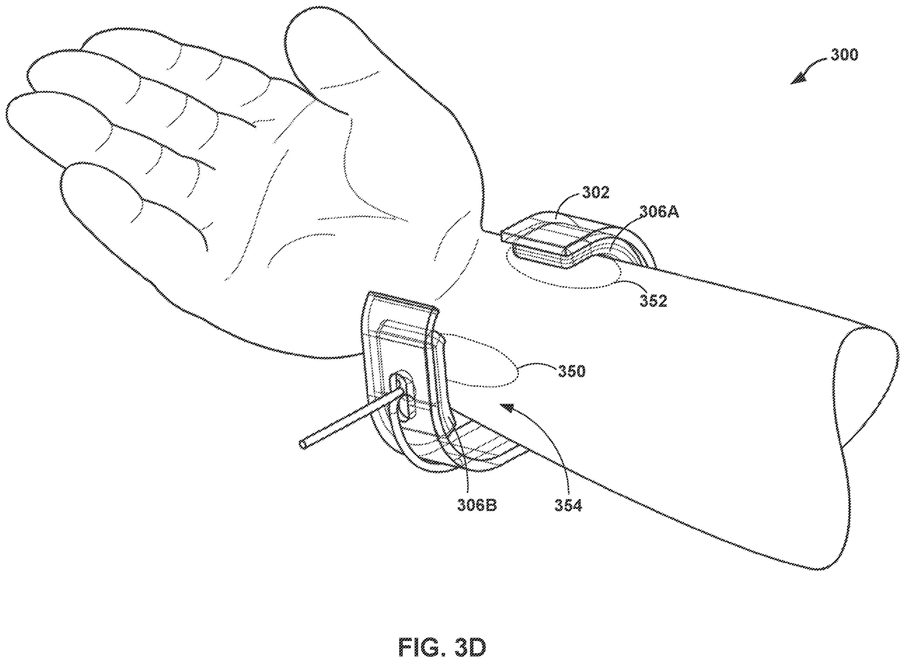

For example, as illustrated in FIG. 3C, in a first orientation, first expandable member 306A may be oriented over ulnar region 350 (indicated by dashed lines) of wrist 354 of the patient and not over radial region 352 (indicated by dashed lines) of wrist 354, and second expandable member 306B may be oriented over radial region 352 and not over ulnar region 350. As illustrated in FIG. 3D, in a second, different (e.g., reversed) orientation, expandable member 306A may be oriented over radial region 352 of wrist 354 of the patient and not over ulnar region 350 of wrist 354, and expandable member 306B may be oriented over ulnar region 350 and not over radial region 352. In this way, as discussed above in reference to FIGS. 2C and 2D, a clinician may change the orientation of non-circumferential tissue compression device 300 during a percutaneous coronary procedure.

In some examples, expandable members 306A, 306B may be fluidly coupled by a connecting tube 334. Fluidly coupling expandable members 306A, 306B with connecting tube 334 may enable expandable members 306A, 306B to be inflated with a single inflation device (e.g., inflation device 130) and/or deflated with a single deflation device (e.g., deflation device 132). In other examples, each of first expandable member 306A and second expandable member 306B are fluidically separate from each other and may be fluidly coupled to respective inflation devices and respective deflation devices. In this way, a pressure of each of first expandable member 306A and second expandable member 306B may be independently controlled. For example, before arteriotomy of the radial artery, first expandable member 306A oriented over ulnar region 350 may be inflated to at least partially occlude the ulnar artery which, as discussed above, may facilitate access at the radial artery. After arteriotomy of the radial artery, e.g., prior to application of pressure to the access site, second expandable member 306B oriented over radial region 352 may be inflated to apply pressure to the access site to achieve patent hemostasis of the access site at the radial artery. In this way, non-circumferential tissue compression device 300 may facilitate both ulnar compression (e.g., to improve accuracy and speed of vasculature access via arteriotomy of the radial artery by making radial pulse stronger and/or enlarging the radial artery to improve success rate of fingertip palpitation-guided access to the radial artery) and radial compression (e.g., to achieve hemostasis at the transradial access site without causing radial artery occlusion) without requiring the clinician to remove non-circumferential tissue compression device 300 from the wrist of the patient.

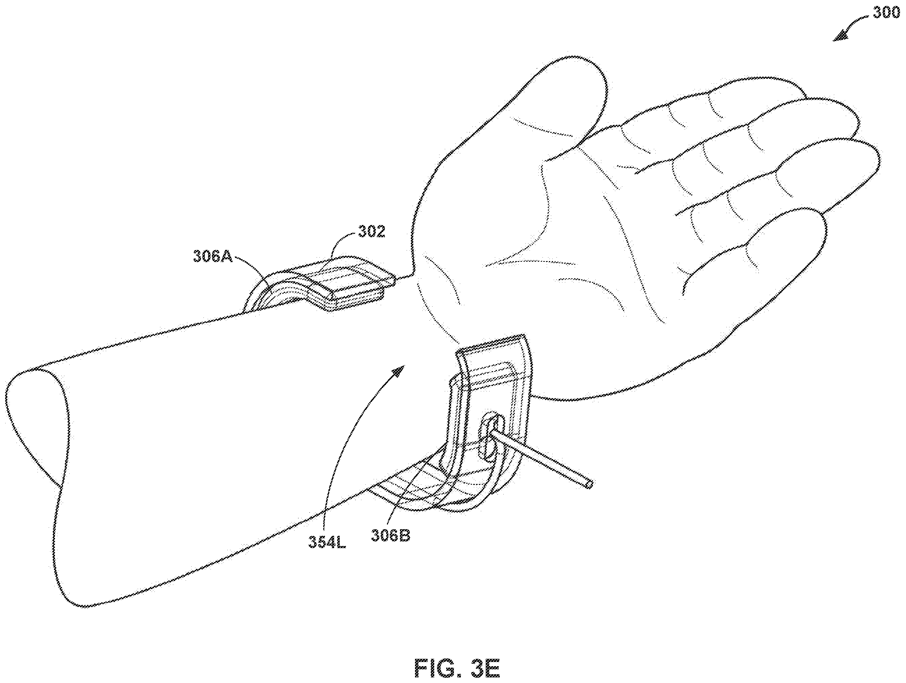

As discussed above in reference to FIG. 1, a non-circumferential tissue compression device may be configured to engage a left wrist of the patient, a right wrist of the patient, or both the left wrist and the right wrist of the patient. For example, as illustrated in FIG. 3E, non-circumferential tissue compression device 300 may be fitted to the left wrist 354L of the patient. In this way, non-circumferential tissue compression device 300 may enable both right and left transradial percutaneous coronary procedures.

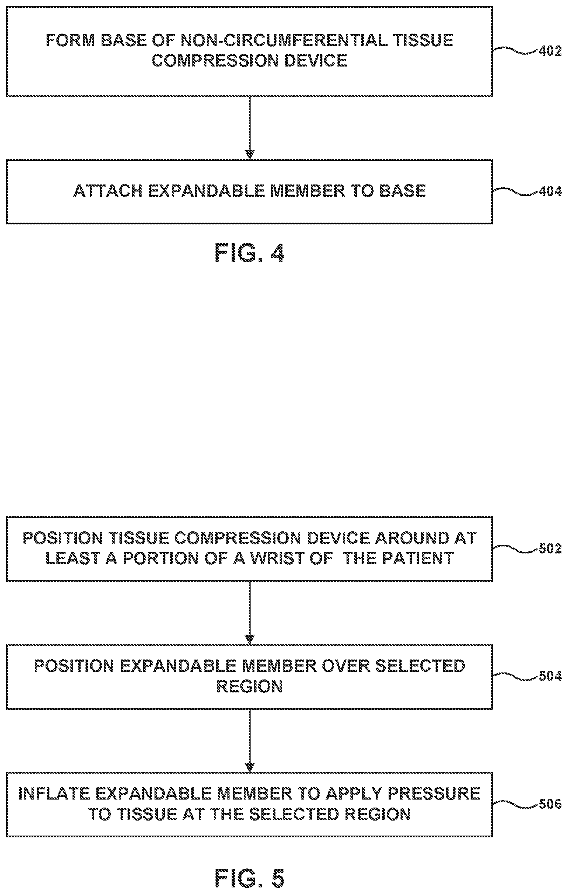

The non-circumferential tissue compression devices described herein may be assembled using any suitable technique. FIG. 4 is a flow diagram illustrating an example method of assembling a non-circumferential tissue compression device including a base and an expandable member. The non-circumferential tissue compression device may be the same as or substantially similar to non-circumferential tissue compression devices 100, 200, and 300 discussed above with respect to FIGS. 1-3E. Although FIG. 4 is described with respect to non-circumferential tissue compression device 100, in other examples, the technique illustrated in FIG. 4 may be used to assemble other non-circumferential tissue compression devices, such as non-circumferential tissue compression device 200 or 300, or other non-circumferential tissue compression devices including a base and an expandable member mechanically connected to the base.

In accordance with the technique illustrated in FIG. 4, a user or a device forms base 102 of non-circumferential tissue compression device 100 (402). Forming base 102 may include thermoforming or molding base 102. Base 102 may include a substantially rigid material, such as a substantially rigid polymer, configured to be thermoformed to substantially conform to an anatomical shape of the wrist a patient or a substantially rigid thermoset plastic configured to be molded to substantially conform to an anatomical shape of the wrist of a patient. By thermoforming or molding base 102 to conform to an anatomical shape of the wrist of a patient, base 102 may be shaped to improve patient comfort, improve tissue compression at a selected area such as an ulnar region or radial region, or both. In examples in which base 102 includes a plurality of sections, each section may be formed together, or formed separately and subsequently connected, e.g., connected by forming a joint material between adjacent sections.

In examples in which base 102 includes a pad disposed on base 102, assembling non-circumferential tissue compression device 100 may include positioning the pad on base 102 such that the pad is configured to engage at least a portion of the dorsal surface of the wrist of the patient to improve patient comfort when the dorsal surface of the wrist is engaged with first surface 104 of base 102.

The technique illustrated in FIG. 4 also may include forming expandable member 106. In some example forming expandable member 106 may include thermoforming a flexible polymer to define the shape of expandable member 106.

The technique illustrated in FIG. 4 also includes attaching expandable member 106 to base 102 (404). For example, expandable member 106 may be attached to base 102 using an adhesive, thermal bonding, welding, or a mechanical fastener, e.g., by a hook-and-loop fastener. In examples in which expandable member 106 is integrally formed with base 102, attaching expandable member 106 to base 102 may include forming a pocket in base 102 that defines expandable member 106.

In some examples, the technique may include fluidly coupling inflation device 130, deflection device 132, or both to expandable member 106.

The tissue compression devices describe herein may be retained on a hand of a patient using any suitable technique. FIG. 5 is a flow diagram illustrating an example method of using a non-circumferential tissue compression tissue compression device including a base and an expandable member. The non-circumferential tissue compression device may be the same as or substantially similar to non-circumferential tissue compression devices 100, 200, and 300 discussed above with respect to FIGS. 1-3E. Although FIG. 5 is described with respect to non-circumferential tissue compression device 100, in other examples, the technique illustrated in FIG. 5 may be used with other non-circumferential tissue compression devices, such as non-circumferential tissue compression device 200 or 300, or other non-circumferential tissue compression devices including a base and an expandable member mechanically connected to the base.

The method shown in FIG. 5 includes positioning non-circumferential tissue compression device 100 around at least a portion of a wrist of a patient (502). In some examples, a user may pass a wrist of the patient through gap 124. In some examples, the user may deform non-circumferential tissue compression device 100 for fitting to the wrist of the patient, e.g., to sufficiently widen gap 124 to fit over the wrist of the patient.

The method of FIG. 5 also includes positioning expandable member 106 over selected region, such as an ulnar region, a radial region, or both the ulnar region and the radial region of the patient (504). For example, after fitting non-circumferential tissue compression device 100 to the wrist, the user may rotate non-circumferential tissue compression device 100 around the wrist to orient expandable member 106 over the selected region. In some examples, expandable member 106 is movable relative to base 102, and a user may detach expandable member 106 from base 102 and then subsequently reattaching expandable member 106 to base 102 at a different location, thereby enabling a user to adjust the position of expandable member 106 relative to base 102 and the wrist of the patient.

The method of FIG. 5 also includes inflating expandable member 106 to apply pressure to tissue at the selected region (506). For example, expandable member 106 includes the bladder such that inflating expandable member 106 includes inflating the bladder of expandable member 106 to cause compression of tissue near the selected region. In some examples, base 102 includes a substantially rigid thermoplastic or a substantially rigid thermoset plastic such that when tissue compression device 100 is retained on the wrist of the patient increasing pressure in expandable member 106 results in increasing pressure on selected tissue at the selected region.

In examples in which tissue compression device 100 includes second expandable member, the technique may include adjusting a position of second expandable member to position second expandable member over a second selected region, such as an ulnar region, a radial region, both the ulnar region and the radial region of the patient, or a transradial access site. The technique also may include, after adjusting a position of second expandable member, inflating second expandable member to apply pressure to second selected region. For example, the second selected region may include a transradial access site, such that inflating the second expandable member may cause patent hemostasis of a vascular access site at the radial artery.

Various examples have been described. Any combination of the described systems, devices, operations, or functions is contemplated. These and other examples are within the scope of the following claims.

## Claims

1. A non-circumferential tissue compression device (100, 200, 300) comprising:
a base (102, 202, 302) configured to, when on a wrist of a patient, surround at least a portion of the wrist in a non-circumferential configuration; and
an expandable member (106, 206, 306) mechanically connected to the base (102, 202, 302), wherein the expandable member (106, 206, 306) is configured to be positioned over an ulnar region or a radial region of the wrist of the patient when the base (102, 202, 302) surrounds the portion of the wrist, and wherein the expandable member (106, 206, 306) is configured to apply pressure to the ulnar region or the radial region,
wherein the base (102, 202, 302) comprises:
a first section (110) extending from a terminal end (116, 316) to a medial end (118), wherein the first section (110) is configured to traverse one of a radial side of the wrist or an ulnar side of the wrist when the base (102, 202, 302) is on the wrist;
a second section (112) extending from a terminal end (120, 320) to a medial end (122), wherein the second section (112) is configured to traverse the other of the radial side of the wrist or the ulnar side of the wrist when the base (102, 202, 302) is on the wrist; and
a third section (114) extending between the medial end (118) of the first section (110) and the medial end (118) of the second section (112);
**characterized in that** the expandable member (106, 206, 306) comprises a protrusion (207) extending from a surface of the expandable member (106, 206, 306) and configured to apply pressure to a tissue near an ulnar artery or a radial artery of the patient.

2. The tissue compression device of claim 1, wherein, when on the wrist of the patient, the tissue compression device (100, 200, 300) is configured to partially surround the wrist in a first orientation and in a second, different orientation, and wherein, when in the first orientation, the expandable member (106, 206, 306) is positioned over the ulnar region, and wherein, when in the second orientation, the expandable member (106, 206, 306) is positioned over the radial region.

3. The tissue compression device of any one of the preceding claims, wherein the expandable member (106, 206, 306) is a first expandable member (306A) configured to be positioned over the ulnar region of the wrist of the patient when the base (102, 202, 302) surrounds the portion of the wrist and apply pressure to the ulnar region, wherein the tissue compression device (100, 200, 300) further comprises a second expandable member (306B) mechanically connected to the base (102, 202, 302), wherein the second expandable member (306B) is configured to be positioned over the radial region of the wrist of the patient and apply pressure to the radial region when the base (102, 202, 302) surrounds the portion of the wrist.

4. The tissue compression device of claim 3, wherein the first expandable member (306A) comprises a first bladder configured to inflate to at least a pressure to cause compression of tissue near an ulnar artery of the patient, and wherein the second expandable member (306B) comprises a second bladder configured to inflate to at least a pressure to cause patent hemostasis of a vascular access site at the radial artery of the patient.

5. The tissue compression device of claim 3, wherein the second expandable member (306B) comprises a bladder configured to inflate to at least a pressure to urge the wrist of the patient toward the first expandable member (306A).

6. The tissue compression device of any one of the preceding claims,
wherein the terminal end (116, 316) of the first section (110) and the terminal end (120, 320) of the second section (112) define a gap (124), wherein the gap is sized to enable the wrist of the patient to pass through the gap (124).

## Patentansprüche

1. Nicht umlaufende Gewebekompressionsvorrichtung (100, 200, 300), umfassend:
eine Basis (102, 202, 302), die konfiguriert ist, um, wenn sie an einem Handgelenk eines Patienten ist, mindestens einen Abschnitt des Handgelenks in einer nicht umlaufenden Konfiguration zu umgeben; und
ein expandierbares Element (106, 206, 306), das mit der Basis (102, 202, 302) mechanisch verbunden ist, wobei das expandierbare Element (106, 206, 306) konfiguriert ist, um über einer ulnaren Region oder einer radialen Region des Handgelenks des Patienten positioniert zu werden, wenn die Basis (102, 202, 302) den Abschnitt des Handgelenks umgibt, und wobei das expandierbare Element (106, 206, 306) konfiguriert ist, um Druck auf die ulnare Region oder die radiale Region auszuüben,
wobei die Basis (102, 202, 302) umfasst:
einen ersten Bereich (110), der sich von einem endständigen Ende (116, 316) zu einem medialen Ende (118) erstreckt, wobei der erste Bereich (110) konfiguriert ist, um eine von einer radialen Seite des Handgelenks oder einer ulnaren Seite des Handgelenks zu durchqueren, wenn die Basis (102, 202, 302) an dem Handgelenk ist;
einen zweiten Bereich (112), der sich von einem endständigem Ende (120, 320) zu einem medialen Ende (122) erstreckt, wobei der zweite Bereich (112) konfiguriert ist, um die andere der radialen Seite des Handgelenks oder der ulnaren Seite des Handgelenks zu durchqueren, wenn die Basis (102, 202, 302) an dem Handgelenk ist; und
einen dritten Bereich (114), der sich zwischen dem medialen Ende (118) des ersten Abschnitts (110) und dem medialen Ende (118) des zweiten Bereichs (112) erstreckt;
**dadurch gekennzeichnet, dass** das expandierbare Element (106, 206, 306) einen Vorsprung (207) umfasst, der sich von einer Oberfläche des expandierbaren Elements (106, 206, 306) erstreckt und konfiguriert ist, um Druck auf ein Gewebe in der Nähe einer ulnaren Arterie oder einer radialen Arterie des Patienten auszuüben.

2. Gewebekompressionsvorrichtung nach Anspruch 1, wobei, wenn sie an dem Handgelenk des Patienten ist, die Gewebekompressionsvorrichtung (100, 200, 300) konfiguriert ist, um das Handgelenk in einer ersten Orientierung und in einer zweiten, unterschiedlichen Orientierung teilweise zu umgeben, und wobei, wenn sie in der ersten Orientierung ist, das expandierbare Element (106, 206, 306) über der ulnaren Region positioniert ist, und wobei, wenn sie in der zweiten Orientierung ist, das expandierbare Element (106, 206, 306) über der radialen Region positioniert ist.

3. Gewebekompressionsvorrichtung nach einem der vorstehenden Ansprüche, wobei das expandierbare Element (106, 206, 306) ein erstes expandierbares Element (306A) ist, das konfiguriert ist, um über der ulnaren Region des Handgelenks des Patienten positioniert zu werden, wenn die Basis (102, 202, 302) den Abschnitt des Handgelenks umgibt, und Druck auf die ulnare Region auszuüben, wobei die Gewebekompressionsvorrichtung (100, 200, 300) ferner ein zweites expandierbares Element (306B) umfasst, das mit der Basis (102, 202, 302) mechanisch verbunden ist, wobei das zweite expandierbare Element (306B) konfiguriert ist, um über der radialen Region des Handgelenks des Patienten positioniert zu werden und Druck auf die radiale Region auszuüben, wenn die Basis (102, 202, 302) den Abschnitt des Handgelenks umgibt.

4. Gewebekompressionsvorrichtung nach Anspruch 3, wobei das erste expandierbare Element (306A) eine erste Blase umfasst, die konfiguriert ist, um sich auf mindestens einen Druck aufzublähen, um eine Kompression von Gewebe in der Nähe einer ulnaren Arterie des Patienten zu bewirken, und wobei das zweite expandierbare Element (306B) eine zweite Blase umfasst, die konfiguriert ist, um sich auf mindestens einen Druck aufzublähen, um eine offene Hämostase einer Gefäßzugangsstelle an der radialen Arterie des Patienten zu bewirken.

5. Gewebekompressionsvorrichtung nach Anspruch 3, wobei das zweite expandierbare Element (306B) eine Blase umfasst, die konfiguriert ist, um sich auf mindestens einen Druck aufzublähen, um das Handgelenk des Patienten in Richtung des ersten expandierbaren Elements (306A) zu drängen.

6. Gewebekompressionsvorrichtung nach einem der vorstehenden Ansprüche,
wobei das endständige Ende (116, 316) des ersten Bereichs (110) und das endständige Ende (120, 320) des zweiten Bereichs (112) einen Spalt (124) definieren, wobei der Spalt bemessen ist, um zu ermöglichen, dass das Handgelenk des Patienten durch den Spalt (124) hindurchtreten kann.

## Revendications

1. Dispositif de compression de tissu non circonférentiel (100, 200, 300) comprenant :
une base (102, 202, 302) conçue pour, lorsqu'elle est sur un poignet d'un patient, entourer au moins une partie du poignet dans une configuration non circonférentielle ; et
un élément expansible (106, 206, 306) relié mécaniquement à la base (102, 202, 302), dans lequel l'élément expansible (106, 206, 306) est conçu pour être positionné sur une région cubitale ou une région radiale du poignet du patient lorsque la base (102, 202, 302) entoure la partie du poignet, et dans lequel l'élément expansible (106, 206, 306) est conçu pour appliquer une pression sur la région cubitale ou la région radiale,
dans lequel la base (102, 202, 302) comprend :
une première section (110) s'étendant d'une extrémité terminale (116, 316) à une extrémité médiane (118), dans lequel la première section (110) est conçue pour traverser l'un parmi un côté radial du poignet ou un côté cubital du poignet lorsque la base (102, 202, 302) est sur le poignet ;
une deuxième section (112) s'étendant d'une extrémité terminale (120, 320) à une extrémité médiane (122), dans lequel la deuxième section (112) est conçue pour traverser l'autre parmi le côté radial du poignet ou le côté cubital du poignet lorsque la base (102, 202, 302) est sur le poignet ; et
une troisième section (114) s'étendant entre l'extrémité médiane (118) de la première section (110) et l'extrémité médiane (118) de la deuxième section (112) ;
**caractérisé en ce que** l'élément expansible (106, 206, 306) comprend une protubérance (207) s'étendant à partir d'une surface de l'élément expansible (106, 206, 306) et conçue pour appliquer une pression sur un tissu proche d'une artère cubitale ou d'une artère radiale du patient.

2. Dispositif de compression de tissu selon la revendication 1, dans lequel, lorsqu'il est sur le poignet du patient, le dispositif de compression de tissu (100, 200, 300) est conçu pour entourer partiellement le poignet dans une première orientation et dans une seconde orientation différente, et dans lequel, lorsqu'il est dans la première orientation, l'élément expansible (106, 206, 306) est positionné sur la région cubitale, et dans lequel, dans la seconde orientation, l'élément expansible (106, 206, 306) est positionné sur la région radiale.

3. Dispositif de compression de tissu selon l'une quelconque des revendications précédentes, dans lequel l'élément expansible (106, 206, 306) est un premier élément expansible (306A) conçu pour être positionné sur la région cubitale du poignet du patient lorsque la base (102, 202, 302) entoure la partie du poignet et appliquer une pression sur la région cubitale, dans lequel le dispositif de compression de tissu (100, 200, 300) comprend en outre un second élément expansible (306B) relié mécaniquement à la base (102, 202, 302), dans lequel le second élément expansible (306B) est conçu pour être positionné sur la région radiale du poignet du patient et appliquer une pression sur la région radiale lorsque la base (102, 202, 302) entoure la partie du poignet.

4. Dispositif de compression de tissu selon la revendication 3, dans lequel le premier élément expansible (306A) comprend une première vessie conçue pour se gonfler à au moins une pression afin de provoquer une compression de tissu proche d'une artère cubitale du patient, et dans lequel le second élément expansible (306B) comprend une seconde vessie conçue pour se gonfler à au moins une pression afin de provoquer une hémostase patente d'un site d'accès vasculaire au niveau de l'artère radiale du patient.

5. Dispositif de compression de tissu selon la revendication 3, dans lequel le second élément expansible (306B) comprend une vessie conçue pour se gonfler à au moins une pression pour pousser le poignet du patient vers le premier élément expansible (306A).

6. Dispositif de compression de tissu selon l'une quelconque des revendications précédentes,
dans lequel l'extrémité terminale (116, 316) de la première section (110) et l'extrémité terminale (120, 320) de la seconde section (112) définissent un espace (124), dans lequel l'espace est dimensionné pour permettre au poignet du patient de passer à travers l'espace (124).
